Europäisches Patentamt

European Patent Office

Office européen des brevets

(19)

(11) **EP 1 479 338 B1**

(12) **FASCICULE DE BREVET EUROPEEN**

(45) Date de publication et mention
de la délivrance du brevet:
**06.12.2006 Bulletin 2006/49**

(51) Int Cl.:
***A61B 5/024*** *(2006.01)*

(21) Numéro de dépôt: **03011524.0**

(22) Date de dépôt: **21.05.2003**

(54) **Instrument portable de mesure d'une grandeur physiologique comprenant un dispositif pour l'illumination de la surface d'un tissu organique**

Tragbares Gerät zur Erfassung einer physiologischen Messgrösse mit einem Gerät zur Beleuchtung der Oberfläche von organischem Gewebe

Portable instrument for measuring a physiological variable comprising a device to illuminate the surface of organic tissue

(84) Etats contractants désignés:
**AT BE BG CH CY CZ DE DK EE ES FI FR GB GR HU IE IT LI LU MC NL PT RO SE SI SK TR**

(43) Date de publication de la demande:
**24.11.2004 Bulletin 2004/48**

(73) Titulaire: **ASULAB S.A.**
**2074 Marin (CH)**

(72) Inventeur: **Gueissaz, François**
**2075 Wavre (CH)**

(74) Mandataire: **Ravenel, Thierry Gérard Louis et al**
**I C B**
**Ingénieurs Conseils en Brevets SA**
**Rue des Sors 7**
**2074 Marin (CH)**

(56) Documents cités:
**US-A- 5 506 929        US-A1- 2002 188 210**

EP 1 479 338 B1

Printed by Jouve, 75001 PARIS (FR)

**Description**

DOMAINE TECHNIQUE

La présente invention concerne de manière générale un instrument portable de mesure d'une grandeur physiologique (par exemple le rythme cardiaque) agencé pour être amené au contact de la surface d'un tissu organique (par ex. la peau) comprenant, disposés essentiellement dans un même plan, un dispositif d'illumination comprenant au moins une source lumineuse de surface d'illumination déterminée pour soumettre une portion dudit tissu organique à une émission lumineuse dans au moins une gamme de longueurs d'onde déterminée, et un dispositif de détection distant du dispositif d'illumination pour détècter l'intensité de l'émission lumineuse produite par le dispositif d'illumination après propagation dans le tissu organique. La présente invention concerne plus particulièrement un tel instrument de mesure susceptible d'être porté au poignet, par exemple sous la forme d'un instrument présentant une configuration analogue à une montre-bracelet.

ARRIÈRE-PLAN TECHNOLOGIQUE

De tels instruments portables de mesure sont déjà connus. Ces dispositifs portables sont notamment employés pour permettre la détection, par voie optique, du rythme cardiaque et/ou du taux d'oxygène dans le sang d'un patient. On les trouve sous des formes variées allant de pinces destinées à être placées sur une zone du corps humain (typiquement sur l'extrémité d'un doigt, sur le lobe de l'oreille ou sur toute autre extrémité du corps humain suffisamment irriguée par le sang) à des dispositifs portés au poignet présentant une allure analogue à celle d'une montre-bracelet.
Dans le cadre d'une application à la mesure du rythme cardiaque, le dispositif d'illumination est utilisé pour produire une illumination adéquate d'une partie du tissu organique (typiquement la peau) et est associé à un ou plusieurs photorécepteurs pour détecter l'intensité de l'émission lumineuse produite par le dispositif d'illumination après propagation dans le tissu organique. Les variations de pulsation du flux sanguin induisent une variation de l'absorption de l'émission lumineuse produite par le dispositif d'illumination, variation d'absorption dont la fréquence correspond essentiellement à la fréquence des pulsations cardiaques. Une détection de l'intensité de l'émission lumineuse après propagation dans le tissu organique accompagnée d'un traitement adéquat du ou des signaux de mesure permet d'extraire une indication du rythme cardiaque. Les dispositifs d'illumination communément employés pour ce type d'application sont relativement simples et consistent typiquement d'une ou plusieurs sources lumineuses quasi-ponctuelles. Il s'agit typiquement de diodes électroluminescentes ou LED ("light-emitting diode") émettant dans une gamme de longueurs d'onde déterminée. Les dispositifs dé détection comportent quant à eux communément un ou plusieurs photorécepteurs constitués par exemple de photodiodes ou phototransistors.
La demande de brevet US 2002/0188210 A1 décrit par exemple un instrument portable de détection du rythme cardiaque destiné à être porté sur la partie interne du poignet, à proximité de l'artère du poignet. Dans cet exemple, il est en particulier proposé de disposer plusieurs photorécepteurs autour d'une unique diode électroluminescente. Alternativement, il est par ailleurs proposé de disposer plusieurs diodes électroluminescentes autour d'un unique photorécepteur, cette alternative n'étant toutefois pas souhaitable en raison de l'accroissement de la consommation en énergie et de l'augmentation proportionnelle des coûts de fabrication et de construction.
Dans tous les cas de figures, la ou les sources lumineuses sont, comme déjà mentionné, des sources quasi-ponctuelles dont le cône ou la surface d'émission ne couvre typiquement qu'une superficie de quelque mm$^2$. Cette surface d'émission ou d'illumination réduite présente un inconvénient pour les applications nécessitant l'illumination d'un tissu organique dans la mesure où ce tissu organique présente pour ainsi dire systématiquement des zones localisées où le degré d'absorption de l'émission lumineuse est fortement différent des zones avoisinantes. Dans le cas d'une illumination de la peau d'un utilisateur par exemple, les grains de beauté, les poils ou autres modifications localisées de l'épiderme peuvent ainsi causer une variation importante de l'absorption de l'émission lumineuse et avoir une influence négative sur la qualité de la mesure de la grandeur physiologique désirée. Les mouvements relatifs éventuels entre le dispositif de mesure et le tissu organique illuminé ajoutent encore à la dégradation de la qualité de la mesure. On comprendra que ces problèmes se posent également en ce qui concerne le dispositif de détection.
Il est donc désirable d'illuminer de manière plus uniforme et sur une superficie plus importante la surface du tissu organique afin de réduire ou minimiser le plus possible l'influence de ces "défauts" localisés du tissu illuminé. Une solution peut consister à multiplier les sources lumineuses pour couvrir une superficie plus importante du tissu organique. Comme déjà mentionné en référence au document US 2002/0188210 A1 ci-dessus, cette solution n'est toutefois pas souhaitable essentiellement pour des raisons de consommation et de coûts. Il convient par ailleurs de constater que l'augmentation du nombre de sources lumineuses engendre par ailleurs des problèmes de connectique et de construction.
Il est alternativement ou en complément désirable de capter l'émission lumineuse émanant du tissu organique après propagation dans ce dernier sur une superficie plus étendue pour minimiser l'influence des défauts localisés sur la qualité de la détection des signaux.

Un but général de la présente invention est donc de proposer un instrument portable de mesure permettant de minimiser les influences de défauts localisés du tissu organique sur la qualité de l'illumination et/ou de la détection de l'émission lumineuse après propagation tout en maintenant une consommation en énergie aussi faible que possible afin de rendre une telle solution mieux adaptée pour des applications portables.

Un autre but de la présente invention est de proposer une solution dont la construction reste relativement simple et dont l'encombrement, en particulier en épaisseur, reste réduit.

Encore un autre but de la présente invention est de proposer une solution qui permette d'optimiser au mieux la surface à disposition sur l'instrument pour assurer une illumination la plus étendue possible du tissu organique et/ou assurer une détection de l'émission lumineuse après propagation dans le tissu organique sur une superficie étendue.

RÉSUMÉ DE L'INVENTION

La présente invention a ainsi pour objet un instrument de mesure d'une grandeur physiologique tel que susmentionné dont les caractéristiques sont énoncées dans les revendications indépendantes 1 et 2.

Des modes de réalisation avantageux de la présente invention font l'objet des revendications dépendantes.

Selon la solution proposée dans la revendication 1, l'instrument portable de mesure est ainsi muni d'un dispositif d'illumination comportant un élément optique formant guide couplé à au moins une source lumineuse pour guider l'émission lumineuse de cette source par réflexion totale interne dans une direction essentiellement parallèle à la surface du tissu organique à illuminer et pour répartir cette émission lumineuse en plusieurs zones déterminées de la surface du tissu organique sur une superficie sensiblement supérieure à la surface d'illumination de la source lumineuse.

Selon la solution alternative de la revendication 2, le dispositif de détection comporte un tel élément optique, cette fois-ci pour capter en plusieurs zones de la surface du tissu organique l'émission lumineuse produite par le dispositif d'illumination après propagation dans le tissu organique et pour guider cette émission lumineuse par réflexion totale interne vers au moins un photorécepteur. Ces alternatives peuvent avantageusement être combinées.

Selon des variantes avantageuses de ces solutions, l'élément optique est configuré pour présenter une structure ramifiée et/ou une structure au moins partiellement cellulaire. Préférablement, les zones d'illumination définies par le guide optique d'illumination sont déterminées pour être à une distance sensiblement constante du point le plus proche du pourtour géométrique du dispositif de détection. Dans le cas où le dispositif de détection est constitué d'un ou plusieurs photorécepteurs quasi-ponctuels, l'élément optique agissant comme guide optique présente avantageusement une structure dont la géométrie coïncide essentiellement avec un arc de cercle ou un cercle de rayon prédéterminé autour de la position de chaque photorécepteur. Une structure présentant essentiellement la forme d'une structure en nid d'abeille est notamment proposée.

Cette solution permet d'optimiser au mieux l'espace à disposition pour assurer une illumination adéquate du tissu organique. L'inventeur a pu constater en effet qu'un élément optique structuré de la sorte était particulièrement adapté pour produire l'illumination requise permettant d'assurer une détection optimale de la grandeur physiologique désirée. Cette solution permet de canaliser de manière judicieuse et optimale le flux lumineux produit par la ou les sources lumineuses afin d'illuminer le tissu organique en un nombre de zones disposées de manière déterminée au voisinage du dispositif de détection. Cette solution favorise en outre une optimisation de l'illumination pour une consommation en énergie donnée. Selon les solutions de l'art antérieur, le flux lumineux des sources lumineuses n'est pour ainsi dire pas canalisé. Il en résulte une illumination non optimale, non seulement en terme de distribution mais également en terme de consommation en énergie, car l'émission lumineuse se propage typiquement dans n'importe quelle direction autour de la source. Selon la présente solution, l'élément optique peut être structuré de sorte que l'émission lumineuse émergeant de l'élément optique illumine le tissu organique sous un angle prédéterminé en direction du dispositif de détection, optimisant ainsi plus encore l'utilisation de l'énergie à disposition.

On notera en particulier que le guide optique d'illumination peut être structuré de manière à assurer que l'émission lumineuse émanant du dispositif d'illumination soit proche d'une verticale au tissu organique à illuminer. Ceci permet avantageusement d'illuminer en profondeur le tissu organique tout en évitant dans la mesure du possible un couplage direct entre les dispositifs d'illumination et de détection. Selon les solutions de l'art antérieur, l'inconvénient des sources lumineuses classiques (notamment des LEDs) réside dans la nature non orientée de l'émission lumineuse produite, émission lumineuse qui se propage ainsi dans toutes les directions dans le tissu organique. L'utilisation d'un guide optique pour l'illumination du tissu organique permet de canaliser l'émission lumineuse pour qu'elle se propage en profondeur dans le tissu. L'utilisation d'un guide optique pour la détection permet de manière analogue de favoriser une détection de l'émission lumineuse provenant des profondeurs du tissu organique illuminé, ceci au détriment de l'émission lumineuse éventuelle qui se propage en surface du tissu organique et qui n'est que faiblement modulée par le flux sanguin.

Selon une variante de réalisation particulièrement souhaitable, l'élément optique est un guide optique plein à structures micro-prismatiques. Plus particulièrement, l'élément optique présente une première face, ou face inférieure, dirigée vers le tissu organique, une seconde face, ou face supérieure, opposée à la première face, et des faces latérales joignant les première et seconde faces, orientées essentiellement perpendiculairement à la surface du tissu organique. Les structures micro-prismatiques sont ménagées sur la seconde face et/ou les faces latérales pour réorienter l'émission lumineuse au travers de la première face en direction du tissu organique à illuminer ou respectivement pour réorienter l'émission lumineuse émanant du tissu organique vers le ou les photorécepteurs associés.

Afin d'assurer une illumination ou une détection aussi uniforme et homogène que possible, la longueur des structures micro-prismatiques et/ou leur nombre augmente préférablement au fur et à mesure que l'on s'éloigne de la source lumineuse, respectivement du photorécepteur associé, sur le chemin optique de l'émission lumineuse. Ces deux alternatives sont applicables indépendamment l'une de l'autre ou en combinaison et présentent l'avantage de compenser la diminution de l'intensité de l'émission lumineuse produite ou captée.

Préférablement, pour l'illumination, chaque structure micro-prismatique présente une première facette orientée dans la direction d'où provient l'émission lumineuse de la source lumineuse et de sorte à rediriger cette émission lumineuse en direction de la première face de l'élément optique. Une structure similaire peut être adoptée pour la détection de l'émission lumineuse émanant du tissu organique.

La première facette au moins de chaque structure micro-prismatique peut être recouverte d'un revêtement réfléchissant afin d'augmenter le flux lumineux utile pour l'illumination ou la détection. Des parties plus étendues des parois de l'élément optique, telles les parois latérales ou !a paroi supérieure de l'élément optique, pourraient être recouvertes d'un revêtement réfléchissant. Il est à noter qu'en effectuant un revêtement total de la surface où sont ménagées les structures micro-prismatiques, on diminue un peu le surcoût de fabrication par rapport à un revêtement sélectif des structures micro-prismatiques uniquement, mais on augmente également les pertes par absorption dues aux surfaces réfléchissantes.

Selon une autre variante, le dispositif d'illumination comporte des première et deuxième sources lumineuses produisant des émissions lumineuses dans des gammes de longueurs d'onde déterminées et distinctes, le guide optique étant agencé pour mélanger les émissions lumineuses de ces première et seconde sources. L'utilisation de deux sources distinctes peut s'imposer dans certaines applications, notamment pour la détection du rythme cardiaque ou la mesure du taux d'oxygène dans le sang, où l'on exploite les différences d'absorption du sang à deux longueurs d'ondes distinctes. Typiquement, des sources émettant dans le rouge et dans l'infrarouge sont utilisées. On peut également exploiter d'autres longueurs d'onde tel dans le jaune (env. 590 nm) ou dans le vert (env. 480-530 nm), le choix dépendant essentiellement de l'application.

Un avantage de cette variante réside dans le fait que l'élément optique joue non seulement le rôle d'un guide optique mais également le rôle d'un "mélangeur" optique permettant de distribuer de manière uniforme sur une même surface des émissions lumineuses selon au moins deux longueurs d'ondes distinctes. Les solutions antérieures utilisant uniquement des sources lumineuses quasi-ponctuelles, telles des LED's, présentent à ce titre un désavantage considérable en ce sens que deux sources distinctes ne peuvent bien évidemment pas être disposées en un même endroit. Il en résulte obligatoirement un décalage spatial entre les sources lumineuses de longueurs d'onde différentes et donc des variations d'absorption potentiellement importantes entre ces deux positions qui peuvent influer négativement sur le résultat de la détection.

Enfin, l'instrument portable de mesure susmentionné est parfaitement adapté pour une utilisation dans un instrument électronique porté au poignet. La solution proposée est particulièrement avantageuse car l'élément optique du dispositif d'illumination présente une construction relativement simple et une faible épaisseur lui permettant d'être incorporé dans le fond de la boîte de l'instrument à proximité de la peau de l'utilisateur.

BRÈVES DESCRIPTIONS DES DESSINS

D'autres caractéristiques et avantages de la présente invention apparaîtront plus clairement à la lecture de la description détaillée qui suit de plusieurs modes de réalisation de l'invention donnés uniquement à titre d'exemples non limitatifs et illustrés par les dessins annexés où :

- la figure 1 montre une vue en plan côté fond d'un instrument électronique portable, selon un mode de réalisation, préférablement destiné à être porté au poignet, pour la mesure d'une grandeur physiologique et comprenant un dispositif d'illumination muni d'un élément optique ramifié et un dispositif de détection muni de trois photorécepteurs quasi-ponctuel disposés autour du dispositif d'illumination ;
- les figures 1a et 1b montrent des vues agrandies respectivement en coupe (selon la ligne A-A de la figure 1b) et en plan d'une partie des ramifications de l'élément optique de la figure 1 où des structures micro-prismatiques sont ménagées sur la face supérieure de l'élément optique; ces vues illustrent par ailleurs l'agencement de la source lumineuse par rapport à l'élément optique du dispositif d'illumination ;
- les figures 2a et 2b montrent des vues analogues aux vues des figures 1a et 1b illustrant une variante de réalisation où les structures micro-prismatiques sont notamment ménagées sur les parois latérales des ramifications ;
- la figure 3 est une vue en plan analogue à celle de la figure 1 illustrant une variante de réalisation où l'élément optique présente des ramifications supplémentaires, ces ramifications définissant avantageusement des cellules ouvertes autour des divers photorécepteurs ;

- la figure 4 illustre encore une autre variante de réalisation où l'élément optique présente des ramifications définissant des cellules fermées de forme polygonale, à l'image d'une structure en nid d'abeille ;
- la figure 5 illustre une variante de réalisation où l'élément optique formant guide présente une structure cellulaire analogue à celle de la figure 4, chaque cellule présentant toutefois une forme essentiellement circulaire ou annulaire ;
- la figure 6 est une vue en perspective schématique d'un instrument électronique destiné à être porté au poignet et se présentant sous une forme analogue à celle d'une montre-bracelet, cet instrument comprenant un dispositif d'illumination selon la variante de la figure 5 disposé dans son fond ;
- la figure 7 illustre une variante de réalisation où le dispositif d'illumination comporte deux guides optiques distincts pour illuminer la surface du tissu organique et où le dispositif de détection lui-même est pourvu de guides optiques pour capter l'émission lumineuse après propagation dans le tissu organique, chaque élément optique formant guide présentant ici la forme d'un barreau essentiellement rectiligne ;
- les figures 8 et 9 illustrent d'autres variantes de réalisation analogues à celles des figures 1 et 3, respectivement, où le dispositif de détection est lui aussi pourvu de guides optiques ; et
- la figure 10 montre un diagramme illustrant schématiquement l'évolution de l'intensité lumineuse d'une source dans le tissu organique, du taux de modulation de l'émission lumineuse et de l'intensité résultante de la modulation en fonction de la distance par rapport à une source lumineuse.

Les divers modes de réalisation qui vont maintenant être présentés sont donnés à titre purement illustratif et non limitatif. En particulier, il convient d'insister à nouveau sur le fait que les modes de réalisation qui vont être présentés peuvent avantageusement mais pas uniquement être implémentés dans un instrument destiné à être porté au poignet. D'autres applications portables de ces solutions sont parfaitement envisageables.

Dans les modes de réalisation illustrés dans les figures, on notera qu'un point commun réside dans l'utilisation d'un dispositif d'illumination comprenant au moins une source lumineuse couplée à un élément optique structuré formant guide, ce guide étant structuré d'une manière déterminée en fonction de la géométrie (en particulier son pourtour) et de la disposition du dispositif de détection. Comme on le verra ultérieurement, le dispositif de détection peut lui aussi être pourvu d'au moins un élément optique formant guide couplé à au moins un photorécepteur. Au titre d'alternative, il est envisageable dans le cadre de la présente invention de munir uniquement le dispositif de détection d'un tel guide optique et d'utiliser une ou plusieurs sources lumineuses conventionnelles. L'utilisation d'un guide optique pour l'illumination est toutefois préférable en raison des gains espérés en termes de consommation et de distribution de l'émission lumineuse.

Il convient de noter que l'on connaît déjà des solutions utilisant des guides optiques pour assurer une illumination quasi-uniforme d'une surface, typiquement la surface d'un support portant des inscriptions ou d'un affichage. La demande de brevet européen EP 1 050 711 au nom de la présente Demanderesse décrit par exemple un dispositif d'illumination orientée d'une surface par un guide muni de structures micro-prismatiques. Ce dispositif d'illumination est utilisé dans le seul but d'illuminer une surface plane, tel le cadran d'une montre, l'émission lumineuse émergeant depuis des faces latérales du guide. L'utilisation d'un tel dispositif d'illumination dans un instrument de mesure d'une grandeur physiologique pour l'illumination d'une portion d'un tissu organique n'est cependant ni décrite, ni suggérée.

Dans le cadre de la présente invention, l'élément optique formant guide sera préférablement un guide optique plein à structures micro-prismatiques, avantageusement ménagées en creux. Le matériau constituant ce guide est préférablement un matériau translucide ou transparent à indice de réfraction n élevée (typiquement compris entre 1,40 et 1,65) choisi parmi les polymères organiques bien connus qui permettent de propager par réflexion totale interne le long du guide au moins une partie de l'énergie lumineuse admise à une extrémité à partir d'une source lumineuse au moins. De tels polymères organiques sont par exemple choisis parmi les polymères acryliques, notamment le polyméthylméthacrylate (PMMA), le polycarbonate et les polyesters.

On notera qu'un milieu diffusant pourrait améliorer l'homogénéité et l'uniformité de l'illumination en cas d'utilisation et de mélange de plusieurs sources lumineuses émettant dans des gammes de longueurs d'onde différentes. On peut par contre s'attendre à des pertes plus importantes en terme d'intensité lumineuse.

La structuration du guide optique pourra être opérée par gravage direct ou indirect, tels qu'un usinage mécanique avec un outil approprié, une attaque à travers des masques en photorésist, par des moyens chimiques ou par des rayons lasers, ces techniques étant citées à titre d'exemples non limitatifs. Il est également possible de structurer le guide optique par réplication à partir d'une matrice gravée qu'on adapte à une machine d'injection pour obtenir les guides par moulage, ou qu'on utilise pour étamper une surface dans laquelle on veut conformer le guide. Quelque soit la technique utilisée, la structuration peut être opérée directement sur le guide ou sur une plaque d'indice de réfraction sensiblement identique à celui du guide qui est par la suite appliquée sur le guide en tant que tel, par exemple par collage.

Comme déjà mentionné plus haut, un revêtement réfléchissant peut être apposé sur certaines facettes du guide optique pour augmenter le flux lumineux utile pour l'illumination du tissu organique.

Concernant la ou les sources lumineuses, celles-ci seront préférablement constituées de sources lumineuses quasi-ponctuelles, telles des diodes électroluminescentes (LED) conventionnelles émettant dans la gamme de longueurs d'onde désirée (par ex. des diodes AlGaAs émettant à 650 nm ou des diodes InGaN émettant à 470 nm, etc.). Le ou les photorécepteurs du dispositif de détection pourront par exemple être constitués de photo-diodes ou photo-transistors présentant une réponse adéquate pour la ou les gammes de longueurs d'onde adoptées.

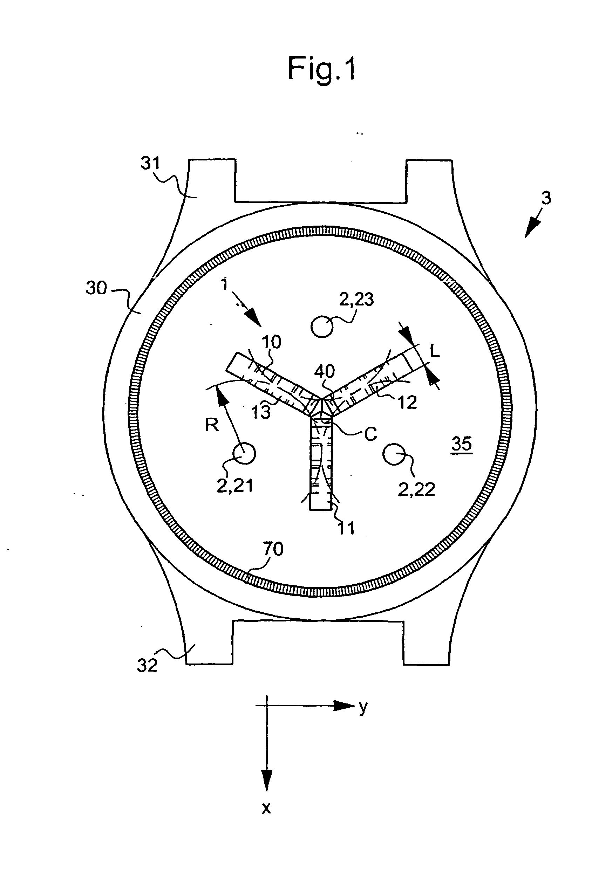

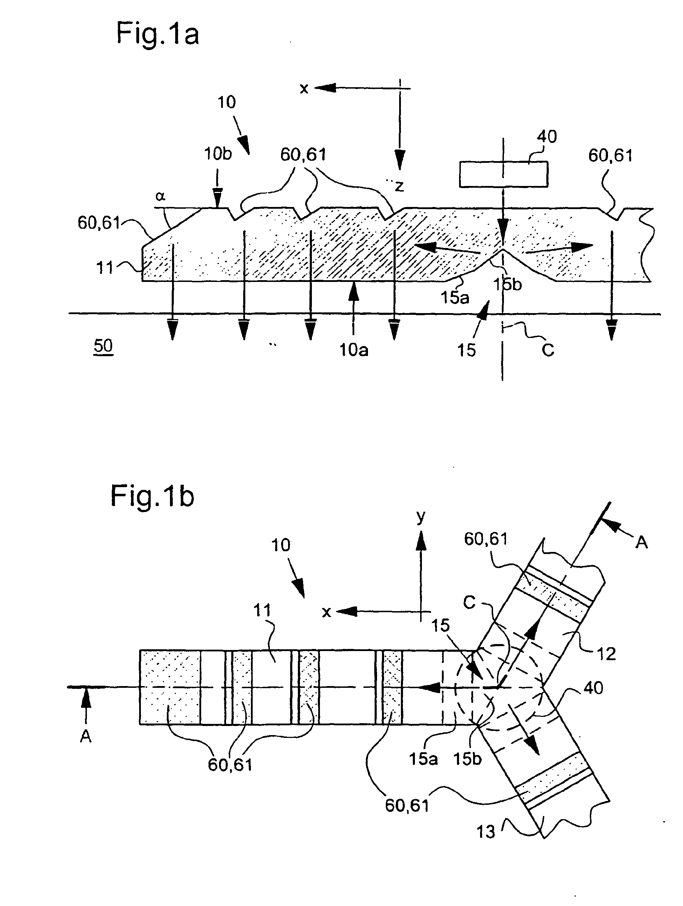

La figure 1 illustre schématiquement un premier mode de réalisation d'un instrument de mesure selon l'invention désigné globalement par la référence numérique 3. Le dispositif d'illumination, désigné par la référence numérique 1, comporte un élément optique 10 formant guide optique couplé à une source lumineuse (au moins) 40 disposée ici sur le centre, désigné C, de la structure et qui n'est que partiellement visible dans la figure 1. Dans l'exemple de !a figure 1, le dispositif d'illumination 1 est associé à un dispositif de détection 2 comprenant trois photorécepteurs 21 à 23 quasi-ponctuels.

L'instrument de mesure de la figure 1 est par exemple destiné à la mesure du rythme cardiaque et présente une allure générale analogue à celle d'une montre-bracelet. Cet instrument 3 comporte ainsi une boîte 30, formant par ailleurs carrure, à l'intérieur de laquelle sont disposés les divers composants électriques et électroniques (non représentés) de l'instrument ainsi qu'un bracelet (non représenté) attaché à la boîte 30 de manière conventionnelle, par exemple par l'intermédiaire de cornes 31, 32 ménagées sur la boîte 30. Les dispositifs d'illumination 1 et de détection 2 sont disposés dans un fond 35 de la boîte de manière à venir en contact avec la peau du porteur. La figure 1 montre à ce titre une vue en plan côté fond de l'instrument 3. Le fond 35 présente en outre préférablement une nervure périphérique, désignée par la référence numérique 70, sur l'utilité de laquelle nous reviendrons ultérieurement plus en détail.

Les dispositifs d'illumination et de détection pourraient alternativement être disposés sur une autre portion adaptée de l'instrument portable, par exemple sur sa face supérieure (dans ce cas, l'utilisateur serait par exemple amené à disposer l'un de ses doigts sur cette face supérieure pour entreprendre la mesure de la grandeur physiologique concernée).

Selon ce premier mode de réalisation, on peut constater que l'élément optique 10 du dispositif d'illumination 1 présente une structure ramifiée en étoile comprenant trois ramifications sensiblement rectilignes 11 à 13 s'étendant essentiellement dans un même plan selon trois directions décalées angulairement de 120° autour du centre C de la structure. Les trois photorécepteurs 21 à 23 du dispositif de détection 2 sont disposés dans cet exemple selon un configuration symétrique sur le bissectrices des angles formés par les trois ramifications 11 à 13, donc également selon trois directions décalées angulairement de 120° autour du centre C de la structure. Les ramifications 11 à 13 et les photorécepteurs 21 à 23 sont ainsi alternés tous les 60° environ autour du centre de la structure.

Plus précisément, les trois photorécepteurs 21 à 23 sont disposés à égale distance du centre C pour former un triangle équilatéral dont les sommets sont constitués par les trois photorécepteurs. Par rapport à ces photorécepteurs, l'élément optique 10 se caractérise par une géométrie ramifiée suivant essentiellement des portions d'arc de cercle de rayon déterminé R autour de la position des photorécepteurs 21 à 23. La distance séparant deux photorécepteurs équivaut ainsi sensiblement à deux fois ce rayon R. Les ramifications 11 à 13 de l'élément optique présentent par ailleurs une largeur moyenne, désigné L, ici constante, dont la valeur est choisie comme une fraction du rayon R.

Le choix de la valeur du rayon R et de la valeur de la largeur moyenne L des ramifications de l'élément optique est dicté par différentes contraintes. L'inventeur a pu constater qu'il convient d'assurer que la distance moyenne séparant chaque photorécepteur des ramifications avoisinantes reste sensiblement constante. Dans cet exemple particulier, cette distance est déterminée d'une part par la valeur du rayon R et par la valeur de la largeur moyenne L des ramifications. Une distance trop faible aurait pour conséquence une trop forte proximité des photorécepteurs 21 à 23 par rapport au dispositif d'illumination 1 ne permettant pas à l'émission lumineuse produite par ce dernier de pénétrer suffisamment profondément dans le tissu organique et d'être suffisamment modulée par les variations du flux sanguin. De même, une distance trop élevée aurait pour conséquence un éloignement trop important des photorécepteurs par rapport au dispositif d'illumination 1 ne permettant pas la détection d'un signal d'intensité suffisante. En pratique, l'inventeur a pu constater qu'une distance de l'ordre de moins d'une dizaine de millimètres était adaptée.

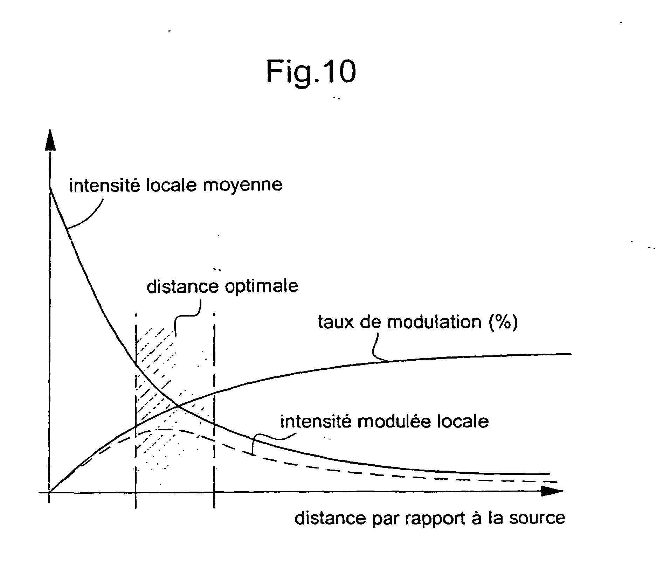

La figure 10 permet de mettre en évidence ce qui vient d'être mentionné. On peut y voir schématiquement que l'intensité locale moyenne décroît de manière quasi exponentielle au fur et à mesure que l'on s'éloigne de la source. D'autre part, le taux de modulation de l'émission lumineuse après propagation dans le tissu organique (émission lumineuse qui est captée par le dispositif de détection) croît à mesure que l'on s'éloigne de la source pour typiquement se stabiliser à partir d'une certaine distance. Cette évolution est liée essentiellement au fait que plus l'on s'éloigne de la source, plus l'émission lumineuse aura pénétré profondément dans le tissu organique illuminé et sera modulée par le flux sanguin. La stabilisation ou saturation du taux de modulation résulte du fait que la répartition des vaisseaux sanguins varie jusqu'à une profondeur déterminée par rapport à la surface du tissu organique à partir de laquelle elle devient homogène. Un chemin de transmission plus long dans les tissus ne contribue plus en conséquence à augmenter le taux de modulation.

L'intensité modulée qui résulte essentiellement de la multiplication des deux courbes susmentionnées (courbe en traits interrompus dans la figure 10) présente ainsi un pic d'amplitude comme schématisé. Pour optimiser le fonctionnement du dispositif optique de mesure, il convient ainsi de sélectionner un écartement adéquat entre les dispositifs d'illumination et de détection qui coïncide essentiellement avec ce pic d'amplitude. Cette distance optimale (indiquée par la partie hachurée dans la figure 10) varie en fonction de la nature du tissu organique illuminé et en particulier de la profondeur à laquelle se trouvent les vaisseaux sanguins. Pour une utilisation au niveau du poignet, l'inventeur a déterminé que cette distance est typiquement inférieure à 10 mm ou moins.

Dans le cas d'une application à un instrument électronique porté au poignet où la surface du fond en contact avec la peau est typiquement de l'ordre d'une dizaine de cm$^2$, on notera qu'il sera possible d'agencer en pratique un peu moins d'une dizaine de photorécepteurs "imbriqués" dans une guide optique structuré tel que proposé.

Dans l'exemple de la figure 1, il convient de constater que la distance entre chaque photorécepteur 21 à 23 et les ramifications avoisinantes n'est pas strictement constante en raison de la forme rectiligne des ramifications 11 à 13. Il convient cependant de préciser qu'un respect strict d'une distance constante entre les photorécepteur et le guide optique n'est pas en soi primordial. Une certaine tolérance est acceptable. Il convient en outre de préciser qu'il est surtout important d'assurer que le guide optique soit structuré pour injecter et distribuer l'émission lumineuse de la ou des sources en des zones déterminées par rapport aux positions des photorécepteurs. Le guide optique lui-même pourrait ainsi suivre une géométrie ne respectant pas nécessairement une distance prédéterminée par rapport à la géométrie du dispositif de détection.

On va maintenant s'intéresser plus particulièrement à la configuration du dispositif d'illumination de la figure 1, configuration qui est détaillée dans les vues des figures 1a et 1b. La figure 1b est une vue agrandie partielle de l'élément optique 10 de la figure 1 vu du côté de sa face supérieure, désignée 10b, c'est-à-dire sa face opposée à la face 10a destinée à être dirigée vers la surface du tissu organique à illuminer (50 dans la figure 1a). La figure 1a quant à elle est une vue en coupe partielle de l'élément optique 10 prise selon la ligne de coupe A-A dans la figure 1b et qui illustre par ailleurs la disposition de la source lumineuse 40.

Par souci de clarté, un référentiel cartésien x-y-z est défini et reporté sur chaque figure pour préciser l'orientation des diverses vues dans l'espace. On notera en particulier que ce référentiel est défini de sorte que le plan général de l'élément optique 10 est parallèle au plan x-y (cf. figure 1), la ramification 11 étant parallèle à l'axe x, et que l'axe z pointe du fond de l'instrument vers la surface du tissu organique à illuminer 50 (cf. figure 1a)

Comme illustré dans les figures 1a et 1b, le dispositif d'illumination 1 comporte dans cet exemple une unique source lumineuse 40, préférablement une diode électroluminescente ou LED, disposée sur le centre C de la structure de sorte que son émission lumineuse soit orientée selon l'axe z vers la partie centrale de l'élément optique comme indiqué par la flèche. Du fait de son orientation perpendiculaire au plan de l'élément optique 10, des facettes 15a, 15b sont ménagées en creux dans la face inférieure 10a de l'élément optique 10. Ces facettes 15a, 15b sont orientées pour rediriger par réflexion totale interne le flux lumineux de la source 40 dans les diverses ramifications de l'élément optique. Dans ce mode de réalisation, ces facettes forment en conséquence un évidement 15 présentant l'allure générale d'un tétraèdre ou plus exactement de deux portions de tétraèdre. Plusieurs facettes (deux dans cet exemple) dont l'angle par rapport au plan x-y décroît au fur et à mesure que l'on s'éloigne du centre C de la structure sont préférablement employées étant donné que l'angle d'incidence des faisceaux produits par la source lumineuse 40 n'est généralement pas constant, les rayons lumineux produits par la source formant typiquement un cône d'émission autour de l'axe z. On comprendra que les facettes 15a, 15b formant l'évidement 15 peuvent être remplacées par toute autre géométrie adéquate permettant de réorienter rémission lumineuse de la source 40 dans les diverses ramifications, par exemple une géométrie de profil courbe.

Comme illustré dans les figures 1a et 1b, l'émission lumineuse produite par la source 40 est ainsi redirigée dans chaque ramification 11, 12, 13 de l'élément optique, chacune de ces ramifications comportant des zones réfléchissantes 60 pour rediriger le flux lumineux par réflexion totale interne en différentes zones de la surface du tissu organique 50 comme schématisé par les flèches dans la figure 1a. Dans l'exemple des figures 1a et 1b, les zones 60 sont ménagées sur la face supérieure 10b de l'élément optique et sont constituées d'au moins une facette 61 inclinée dans la direction d'où provient le faisceau lumineux produit par la source 40 pour former des structures micro-prismatiques. Dans les figures 1a et 1b, on peut voir quatre facettes de ce type sur la ramification 11. Trois d'entre elles sont distribuées sur la longueur de la ramification 11 est sont formées chacune par une partie d'un creux en forme de "V" ménagé sur la face 10b. La quatrième facette est ménagée à l'extrémité de la ramification 11 et présente ici une superficie plus importante que les trois autres.

L'angle des facettes 61 par rapport au plan x-y, désigné $\alpha$, est déterminé de manière à rediriger une partie des faisceaux lumineux incidents vers le tissu organique à illuminer 50. La valeur de cet angle $\alpha$ est essentiellement dictée par la nature du matériau utilisé (notamment l'indice de réfraction n du matériau formant le guide optique 10), l'orientation des faisceaux incidents dans le guide ainsi que la nature du milieu extérieur. Comme déjà mentionné, il est envisageable de revêtir les facettes 61 d'un revêtement réfléchissant (voire la totalité de la face supérieure 10b) pour augmenter le flux lumineux émergeant par la face inférieure 10a de l'élément optique 10.

D'une manière générale, la conception de l'élément optique doit répondre à un certain nombre de contraintes géométriques. L'une d'entre elles est la valeur de l'angle critique désigné $\theta_c$, défini par rapport à la normale à la face concernée, au dessus duquel il se produit une réflexion totale des faisceaux lumineux frappant la face. Cet angle critique est communément donné par la formule suivante :

$$\theta_c = \arcsin(\frac{n_1}{n_2}) \tag{1}$$

où $n_1$ correspond à l'indice de réfraction du milieu externe (égal à 1 dans le cas de l'air) et $n_2$ correspond à l'indice de réfraction du guide où se propagent les faisceaux lumineux. Pour assurer, qu'il se produit toujours une réflexion totale des faisceaux à l'intérieur du guide optique 10, il convient donc de respecter la relation selon laquelle les faisceaux lumineux à l'intérieur du guide doivent présenter un angle d'incidence par rapport à la face concernée qui soit supérieur à l'angle critique $\theta_c$ susmentionné. Dans le cas contraire, une partie des faisceaux lumineux se propagera alors en dehors du guide optique par réfraction. Comme déjà mentionné, des revêtements réfléchissants permettent le cas échéant de réfléchir totalement un faisceau lumineux incident. Il en résulte cependant des pertes par absorption qu'il est préférable de minimiser dans la mesure du possible.

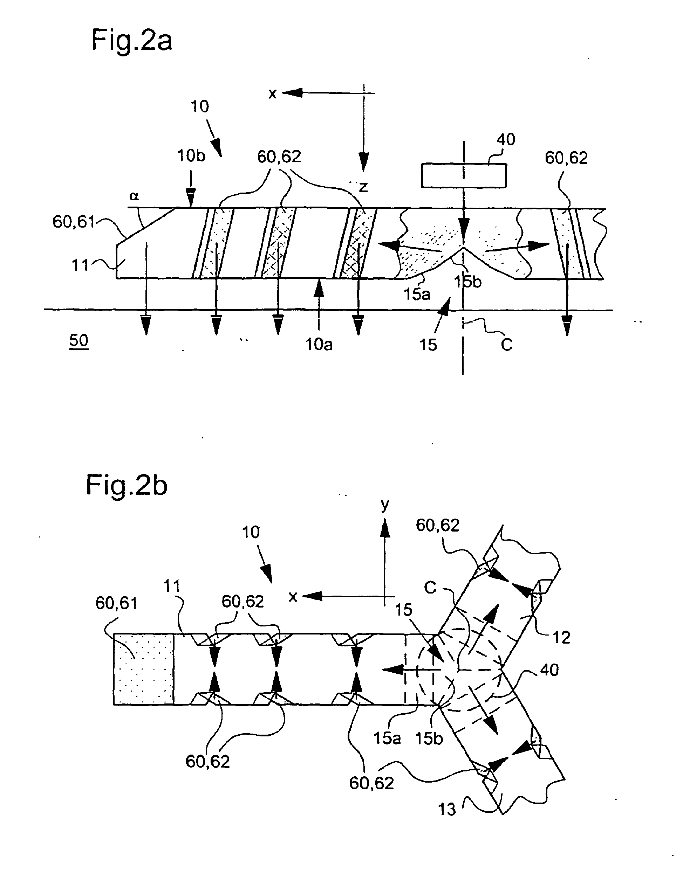

Les figures 2a et 2b montrent une variante de réalisation illustrant une alternative pour la réalisation des zones réfléchissantes 60. Au lieu de réaliser ces zones 60 sur la face supérieure 10b de l'élément optique 10, il est ainsi possible de réaliser ces zones sur les faces latérales de l'élément optique 10 qui sont à la fois essentiellement perpendiculaires à la surface du tissu organique à illuminer et parallèles à la direction générale de propagation du flux lumineux dans l'élément optique. Il s'agit par exemple des faces désignées 10c et 10d de la ramification 11 dans les figures 2a et 2b (et les faces latérales correspondantes des deux autres ramifications 12 et 13). Pour exploiter ces faces, il convient alors, comme illustré dans les figures 2a et 2b, de ménager des facettes, désignées 62, inclinées à la fois dans la direction d'où provient le faisceau lumineux produit par la source 40 ainsi qu'en direction de la surface du tissu organique à illuminer. L'une et/ou l'autre des faces 10c, 10d peut être structurée de la sorte. Il convient de noter que le flux lumineux émergeant par la face 10a de l'élément optique qui est réfléchi par une facette 62 aura une direction générale présentant non seulement une composante selon l'axe z mais également une composante selon l'axe y (voire x). Dans l'exemple discuté, étant donné que des photorécepteurs sont disposés de part et d'autre de chacune des ramifications 11 à 13, il est préférable de structurer de manière symétrique les deux faces latérales de chaque ramification pour assurer une illumination uniforme en distribution.

Les deux méthodes de structuration discutées en référence aux figures 1a, 1b et 2a, 2b peuvent avantageusement être combinées. D'ailleurs, comme illustré dans les figures 2a et 2b, l'extrémité de chaque ramification comporte une facette inclinée 61 à l'image de la solution des figures 1a et 1b. De plus, la taille des facettes (profondeur et/ou largeur) ainsi que leur nombre par unité de surface peut être variable. Il est en particulier souhaitable d'accroître la surface réfléchissante des zones 60 et/ou d'en accroître le nombre au fur et à mesure que l'on s'éloigne de la source lumineuse 40 sur le chemin de propagation du faisceau lumineux, ceci afin de compenser dans une certaine mesure la diminution progressive de l'intensité du flux lumineux.

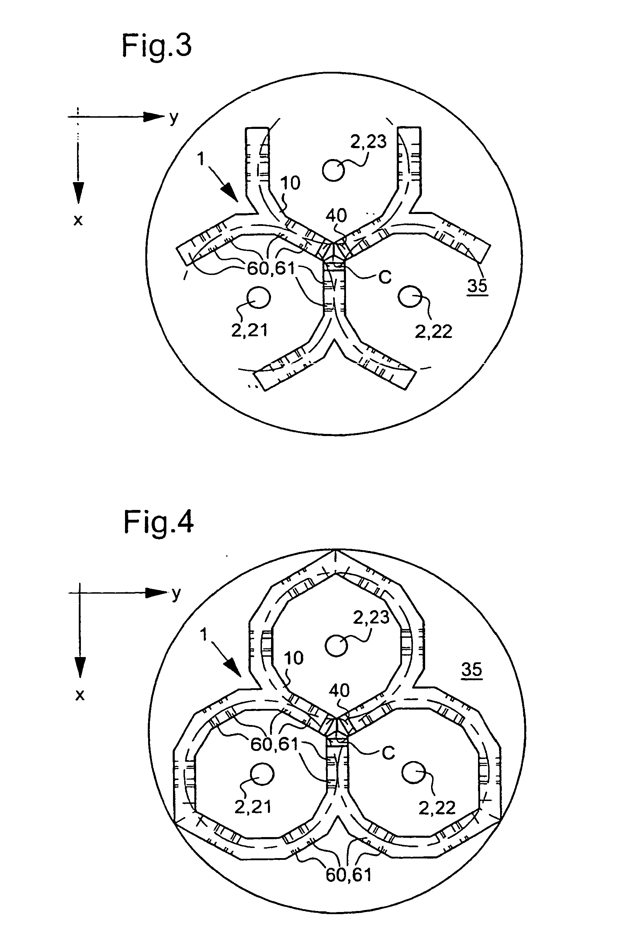

La structure de l'élément optique 10 n'est bien évidemment pas limitée au seul exemple de la figure 1. Comme, illustré dans les figures 3 et 4, l'élément optique 10 peut présenter une structure ramifiée plus étendue formant des cellules ouvertes (figure 3) ou fermées (figure 4) autour de la position des divers photorécepteurs. Pour optimiser au mieux la surface à disposition sur l'instrument portable, une structure cellulaire en nid d'abeille apparaît comme particulièrement avantageuse en raison de son utilisation optimale de l'espace. Comme déjà mentionné plus haut, en adoptant une telle configuration, il apparaît en pratique possible d'agencer moins d'une dizaine de photorécepteurs quasi-ponctuels disposés au sein d'un élément optique cellulaire structuré sur une superficie d'une dizaine de $cm^2$ telle que typiquement disponible sur le fond d'un instrument porté au poignet.

Dans les variantes des figures 3 et 4, on notera que l'on a représenté les zones réfléchissantes 60 de l'élément optique 10 qui permettent la réorientation du flux lumineux en direction du tissu organique à illuminer à l'image des zones réfléchissantes illustrées dans les figures 1, 1a et 1b. Ces représentations ne sont que schématiques et illustratives. En particulier, des structurations du type de celles illustrées au titre de variante dans les figures 2a et 2b peuvent être utilisées en remplacement ou en complément. De plus, le nombre, la forme et la disposition des zones réfléchissantes peut varier. Enfin, les dimensions des zones réfléchissantes (largeur et profondeur) ne sont pas nécessairement à l'échelle.

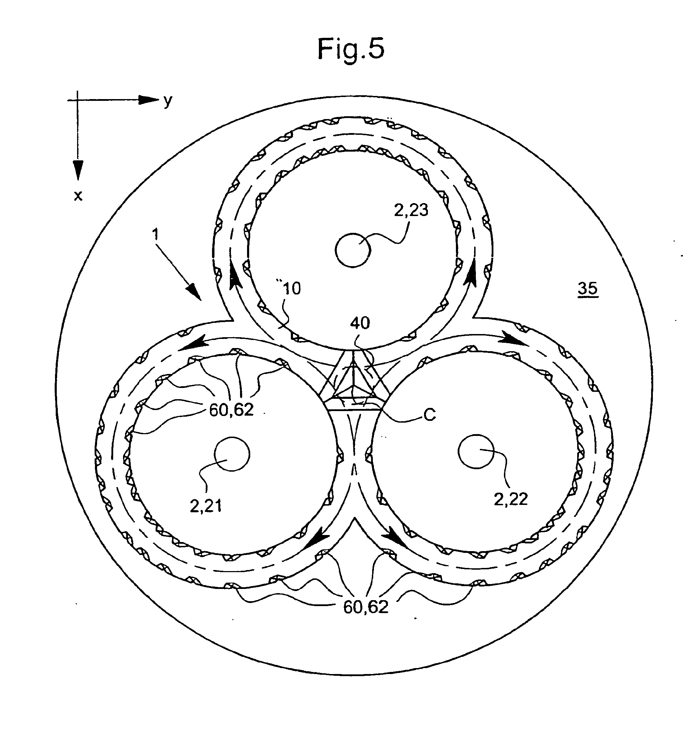

La figure 5 présente encore une autre variante, analogue à celle de la figure 4, où l'élément optique présente une structure cellulaire où chaque cellule présente une forme circulaire ou annulaire en lieu et place de la structure cellulaire polygonale de la figure 4. A titre d'exemple, les zones réfléchissantes 60 sont structurées à l'image des zones des figures 2a et 2b sur les faces latérales du guide 10. Dans la figure 5, on a également indiqué par des flèches le chemin de propagation du faisceau lumineux (toujours produit au centre C de la structure) dans les diverses ramifications de l'élément optique 10.

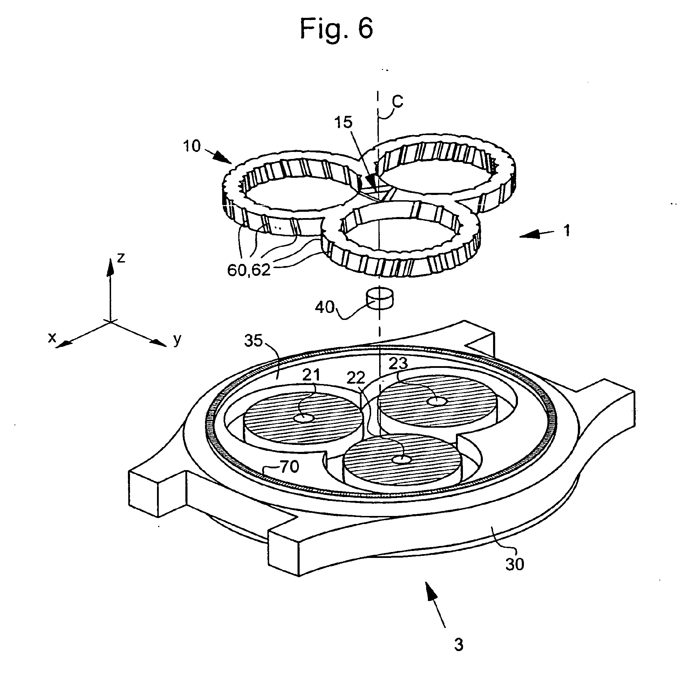

La figure 6 est une vue en perspective illustrant le montage du dispositif d'illumination représenté à la figure 5 dans le fond 35 de l'instrument portable 3. Conformément à ce qui a déjà été présenté, la diode électroluminescente 40 est disposée, dans cet exemple, dans une région centrale du fond 35 et dirigée de sorte que son émission lumineuse soit orientée parallèlement à l'axe z en direction de l'évidement 15 ménagé sur la face inférieure (face dirigée vers le haut dans la figure 6) de l'élément optique 10.

Des espaceurs (non représentés) peuvent être disposés de place en place entre l'élément optique 10 et le fond 35 de manière à ménager un petit d'espace d'air entre les parois du guide 10 et les parois avoisinantes du fond 35. Une paroi intermédiaire (non représentée) pourrait par ailleurs être interposée entre le fond 35 et le guide 10, d'une part, et la surface du tissu organique à illuminer d'autre part. A ce titre, comme déjà mentionné, le fond 35 présente préférablement une nervure périphérique 70, ici de forme annulaire, qui se projette hors du fond en direction du tissu organique à illuminer. Dans cette configuration, cette nervure 70 a essentiellement pour but de répondre à deux objectifs. Le premier de ces objectifs vise à constituer une barrière optique empêchant l'illumination ambiante de venir perturber la mesure de la grandeur physiologique. Le second de ces objectifs vise à assurer que le guide optique ne soit pas directement en contact avec le tissu organique. Il est en effet préférable d'assurer qu'un espace existe en permanence entre le guide optique et le tissu organique illuminé de sorte que la nature de l'interface entre le guide et l'environnement extérieur reste constante et invariable dans le temps, notamment lors de mouvements relatifs de l'instrument portable par rapport au tissu organique.

Dans l'illustration de la figure 6, on notera que la nervure 70 pourrait avantageusement suivre le contour externe du dispositif d'illumination 1, auquel cas cette nervure 70 présenterait une forme à trois lobes plutôt qu'annulaire comme illustré. D'autre part, des nervures pourraient avantageusement être interposées entre les dispositifs d'illumination et de détection pour limiter les couplages directs de lumière entre ces dispositifs.

Outre la présence de la nervure 70, il est par ailleurs préférable de ménager les surfaces du fond 35 qui sont situées entre les dispositifs d'illumination et de détection (les trois surfaces annulaires hachurées dans la figure 6) de sorte qu'elles ne réfléchissent pas l'émission lumineuse produite par le dispositif d'illumination. En effet, dans la mesure où un espace existe entre le fond 35 et le tissu organique sur lequel il repose, une partie de l'émission lumineuse pourrait être réfléchie entre la surface du fond 35 et la surface du tissu organique illuminé avant d'être captée par le dispositif de détection. En ménageant un revêtement anti-réfléchissant sur les surfaces intermédiaires du fond 35, entre les dispositifs d'illumination et de détection, on limitera l'influence parasite de ces réflexions multiples entre le fond et le tissu organique.

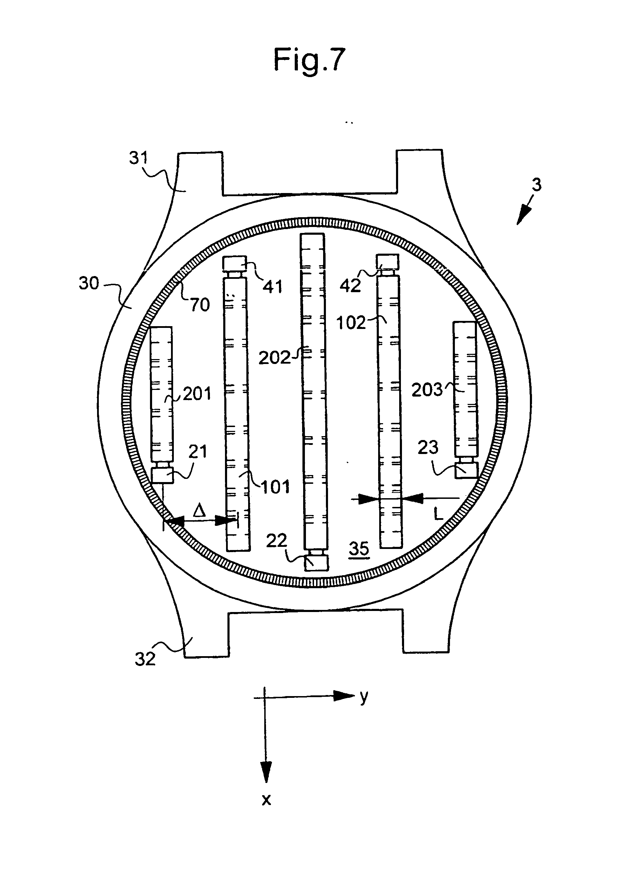

La figure 7 présente encore une autre variante de réalisation qui illustre plusieurs modifications pouvant être envisagées dans le cadre de la présente invention.

En premier lieu, les modes de réalisation présentés plus haut ne comportent qu'un unique élément optique formant guide couplé à une unique source lumineuse. Il est parfaitement envisageable d'utiliser plusieurs guides optiques distincts couplés chacun à une ou plusieurs sources lumineuses. La figure 7 montre par exemple une variante comportant deux guides optiques 101 et 102, de forme essentiellement rectiligne, couplés chacun à une source lumineuse 41, 42.

En second lieu, la disposition de la source lumineuse par rapport au guide optique auquel elle est couplée peut suivre différentes configuration. La figure 7 montre par exemple que chaque source lumineuse 41, 42 est placée en regard d'une extrémité du guide optique associé, l'émission lumineuse étant orientée selon l'axe x dans cet exemple. Cette solution ne nécessite ainsi pas de dispositif optique pour réorienter les faisceaux lumineux dans le plan général du guide comme dans les modes de réalisation des figures 1 à 6.

En troisième lieu, le dispositif de détection peut avantageusement lui aussi être pourvu d'un ou plusieurs éléments optiques formant guide pour capter l'émission lumineuse propagée en différentes zones de la surface du tissu organique examiné, chaque élément optique étant couplé à au moins un photorécepteur. Dans la figure 7, ceci est illustré par la présence de trois éléments optiques 201, 202, 203 formant guide disposés en alternance de part et d'autre des éléments optiques d'illumination 101, 102 et couplés chacun, par l'une de leurs extrémités, à un photorécepteur 21, 22, resp. 23. La structuration de ces éléments optiques est essentiellement similaire à celle des guides optiques d'illumination présentés plus haut.

En quatrième lieu, la géométrie du guide ne doit pas nécessairement suivre des arcs de cercle comme dans les modes de réalisation précédents. La forme en arc de cercle est essentiellement dictée par la géométrie quasi-ponctuelle du ou des photorécepteurs utilisés dans les modes de réalisation des figures 1 à 6. L'essentiel réside surtout dans le respect d'une distance déterminée, dans une gamme de tolérance donnée, entre les zones où le dispositif d'illumination injecte le faisceau lumineux et les zones où le dispositif de détection capte ce faisceau lumineux après propagation dans le tissu organique.

Sur ce dernier point, il convient de constater que l'utilisation d'un ou plusieurs guides optiques pour capter l'émission lumineuse après propagation dans le tissu organique ouvre d'autres possibilités de configuration. Dans la figure 7, on peut ainsi constater que l'utilisation de guides optiques pour la détection de l'émission lumineuse permet de configurer les dispositifs d'illumination et de détection d'une manière particulièrement simple, géométriquement parlant. En effet, les divers guides optiques utilisés 101, 102, 201, 202 et 203 présentent ici simplement la forme d'un barreau structuré rectiligne, donc particulièrement aisé à fabriquer. On veillera dans ce cas à respecter un certain écartement Δ entre les éléments optiques et une certaine largeur L des éléments optiques, cet écartement Δ et cette largeur L pouvant toutefois varier d'un guide optique à l'autre.

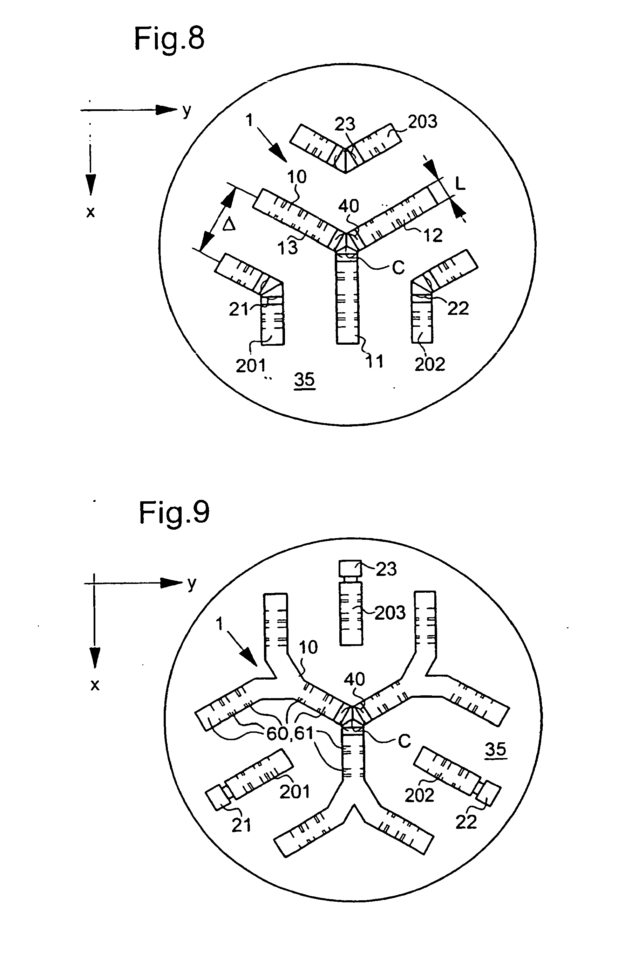

D'autres variantes sont bien évidemment envisageables. Ainsi, il serait envisageable de structurer les éléments optiques de sorte qu'ils présentent des formes complémentaires pouvant être imbriquées l'une dans l'autre (par exemple des structures en peigne ou en spirale, etc.), toujours en respectant une distance essentiellement déterminée entre les zones d'émergence de l'émission lumineuse et les zones de détection de cette émission lumineuse. Les figures 8 et 9 présentent à ce titre des variantes de réalisation analogues à celles illustrées dans les figures 1 et 3, respectivement, où les trois photorécepteur 21, 22, 23 sont couplés à des guides optiques respectifs 201, 202 et 203. A l'image de la figure 7, la variante de la figure 9 montre à nouveau que les photorécepteurs peuvent être couplés à une extrémité des guides optiques associés.

Dans les illustrations des figures 7, 8 et 9, on comprendra que les divers guides optiques d'un même dispositif (illumination ou détection) pourraient être combinés en un seul est même guide optique et que le nombre de sources lumineuses ou des photorécepteurs associés pourrait être réduit.

En dernier lieu, on comprendra par ailleurs que plusieurs sources lumineuses émettant dans des gammes de longueurs d'onde distinctes pourraient être couplées simultanément sur un même guide optique. Dans ce cas, il apparaîtra judicieux de munir le dispositif d'illumination d'un dispositif optique pour mélanger les émissions lumineuses des diverses sources en entrée de l'élément optique ou de coupler ces diverses sources lumineuses de sorte que leur émission lumineuses se mélangent dans le guide optique, l'idée étant surtout que l'émission lumineuse émergeant des différentes zones du guide optique présente une bonne uniformité et homogénéité entre les diverses composantes de longueurs d'onde désirées. Comme déjà mentionné en préambule, l'utilisation d'un milieu diffusant pour la réalisation du guide optique favoriserait une telle homogénéisation, des pertes d'intensité lumineuse étant cependant attendues.

D'une manière générale, on aura compris que l'utilisation d'un élément optique formant guide, tel que proposé ci-dessus, pour illuminer le tissu organique et, le cas échéant, pour détecter l'émission lumineuse après propagation dans le tissu permet d'optimiser au mieux la surface à disposition sur l'instrument portable ainsi que l'utilisation de l'énergie lumineuse disponible.

On comprendra finalement que diverses autres modifications et/ou améliorations évidentes pour l'homme du métier peuvent être apportées aux modes de réalisation décrits dans la présente description sans sortir du cadre de l'invention défini par les revendications annexées. En particulier, la présente invention n'est pas limitée uniquement à une utilisation dans une montre-bracelet mais s'applique à toute autre application portable, au poignet ou non.

**Revendications**

1.  Instrument portable de mesure d'une grandeur physiologique (3) agencé pour être amené au contact de la surface d'un tissu organique (50) comprenant, disposés essentiellement dans un même plan :

    un dispositif d'illumination (1) comprenant au moins une source lumineuse (40; 41, 42) de surface d'illumination déterminée pour soumettre une portion dudit tissu organique à une émission lumineuse dans au moins une gamme de longueurs d'onde déterminée ; et
    un dispositif de détection (2) distant dudit dispositif d'illumination (1) pour détecter l'intensité de l'émission lumineuse produite par ledit dispositif d'illumination après propagation dans ledit tissu organique (50), **caractérisé en ce que** ledit dispositif d'illumination (1) comporte un élément optique formant guide (10; 101, 102) couplé à ladite au moins une source lumineuse (40; 41, 42) pour guider l'émission lumineuse de cette source par réflexion totale interne dans une direction essentiellement parallèle à la surface du tissu organique à illuminer (50) et pour répartir cette émission lumineuse en plusieurs zones d'illumination déterminées sur la surface dudit tissu organique (50) sur une superficie sensiblement plus étendue que la surface d'illumination de ladite source lumineuse (40; 41, 42).

2.  Instrument portable de mesure d'une grandeur physiologique (3) agencé pour être amené au contact de la surface d'un tissu organique (50) comprenant, disposés essentiellement dans un même plan :

    un dispositif d'illumination (1) comprenant au moins une source lumineuse (40; 41, 42) de surface d'illumination déterminée pour soumettre une portion dudit tissu organique à une émission lumineuse dans au moins une gamme de longueurs d'onde déterminée ; et
    un dispositif de détection (2) distant dudit dispositif d'illumination (1) pour détecter l'intensité de l'émission lumineuse produite par ledit dispositif d'illumination après propagation dans ledit tissu organique (50),

**caractérisé en ce que** ledit dispositif de détection (2) comporte un élément optique formant guide (201, 202, 203) couplé à au moins un photorécepteur (21, 22, 23) pour capter en plusieurs zones de la surface du tissu organique (50) l'émission lumineuse produite par le dispositif d'illumination (1) après propagation dans le tissu organique et pour guider cette émission lumineuse par réflexion totale interne vers ledit au moins un photorécepteur (21, 22, 23).

**3.** Instrument selon la revendication 1, **caractérisé en ce que** ledit dispositif de détection (2) présente un pourtour géométrique déterminé et **en ce que** chaque zone d'illumination est déterminée pour être à une distance sensiblement constante du point le plus proche dudit pourtour géométrique du dispositif de détection.

**4.** Instrument selon la revendication 1, 2 ou 3, **caractérisé en ce que** ledit élément optique formant guide présente une structure ramifiée et/ou au moins partiellement cellulaire.

**5.** Instrument selon la revendication 4, **caractérisé en ce que** ledit élément optique formant guide présente essentiellement une structure en nid d'abeille.

**6.** Instrument selon la revendication 1, **caractérisé en ce que** ledit dispositif de détection (2) comporte un ou plusieurs photorécepteurs quasi-ponctuels (21, 22, 23) disposés essentiellement dans le même plan que ledit élément optique formant guide (10) et **en ce que** ledit élément optique formant guide (10) présente une structure dont la géométrie coïncide essentiellement avec un arc de cercle ou un cercle de rayon prédéterminé autour de la position de chaque photorécepteur.

**7.** Instrument selon la revendication 1, **caractérisé en ce que** ledit élément optique (10; 101, 102) est un guide optique plein muni de structures micro-prismatiques réfléchissantes (60) pour réorienter l'émission lumineuse produite par ladite source lumineuse (40; 41, 42) en direction de la surface du tissu organique à illuminer (50),
ledit élément optique présentant une première face (10a), ou face inférieure, dirigée vers ledit tissu organique à illuminer (50), une seconde face (10b), ou face supérieure, opposée à ladite première face, et des faces latérales (10c, 10d) joignant lesdites première et seconde faces, orientées essentiellement perpendiculairement à la surface du tissu organique à illuminer,
chacune desdites structures micro-prismatiques (60) comportant au moins une première facette (61; 62) ménagée sur ladite seconde face (10b) et/ou lesdits faces latérales (10c, 10d) pour réorienter ladite émission lumineuse au travers de ladite première face (10a) en direction dudit tissu organique à illuminer (50).

**8.** Instrument selon la revendication 2, **caractérisé en ce que** ledit élément optique (201, 202, 203) est un guide optique plein muni de structures micro-prismatiques réfléchissantes pour réorienter l'émission lumineuse émanant du tissu organique (50) en direction du ou des photorécepteurs associés (21, 22, 23),
ledit élément optique présentant une première face, ou face inférieure, dirigée vers ledit tissu organique d'où provient l'émission lumineuse (50), une seconde face, ou face supérieure, opposée à ladite première face, et des faces latérales joignant lesdites première et seconde faces, orientées essentiellement perpendiculairement à la surface du tissu organique,
chacune desdites structures micro-prismatiques comportant au moins une première facette ménagée sur ladite seconde face et/ou lesdits faces latérales pour réorienter l'émission lumineuse émanant du tissu organique en direction du ou des photorécepteurs associés.

**9.** Instrument selon la revendication 7 ou 8, **caractérisé en ce que** la première facette au moins de chaque structure micro-prismatique réfléchissante est recouverte d'un revêtement réfléchissant.

**10.** Instrument selon l'une quelconque des revendications 7 à 9, **caractérisé en ce que** la longueur desdites structures micro-prismatiques et/ou leur nombre augmente au fur et à mesure que l'on s'éloigne de ladite source lumineuse, respectivement du photorécepteur associé, sur le chemin optique de l'émission lumineuse dans ledit élément optique.

**11.** Instrument selon la revendication 1, **caractérisé en ce qu'**il comporte une première source lumineuse pour produire une émission lumineuse dans une première gamme de longueurs d'onde déterminée et une seconde source lumineuse pour produire une émission lumineuse dans une seconde gamme de longueurs d'onde déterminée distincte de la première,
ledit élément optique formant guide étant agencé pour mélanger les émissions lumineuses desdites première et seconde sources lumineuses.

**12.** Instrument selon la revendication 1, **caractérisé en ce que** ledit dispositif de détection (2) comporte un élément optique formant guide (201, 202, 203) couplé à au moins un photorécepteur (21, 22, 23) pour capter en plusieurs zones de la surface du tissu organique (50) l'émission lumineuse produite par le dispositif d'illumination (1) après propagation dans le tissu organique et pour guider cette émission lumineuse par réflexion totale interne vers ledit au moins un photorécepteur (21, 22, 23).

**13.** Instrument selon l'une quelconque des revendications précédentes, **caractérisé en ce qu**'il est porté au poignet et comporte une boîte (30) munie d'un fond (35) agencé pour venir au contact de la peau du porteur et dans lequel sont disposés lesdits dispositifs d'illumination (1) et de détection (2).

**14.** Instrument selon la revendication 13, **caractérisé en ce que** ledit fond (35) est pourvu d'une nervure (70) destinée à venir en appui sur ledit tissu organique (50) pour maintenir un espace entre l'élément optique formant guide (10; 101, 102; 201, 202, 203) et le tissu organique.

**15.** Instrument selon la revendication 13, **caractérisé en ce que** au moins une partie de la surface dudit fond (35) située entre les dispositifs d'illumination et de détection est ménagée pour ne pas réfléchir l'émission lumineuse produite par ledit dispositif d'illumination.

**Claims**

**1.** Portable instrument for measuring a physiological quantity (3) arranged to be brought into contact with the surface of an organic tissue (50) including, essentially arranged in a same plane:

an illumination device (1) including at least one light source (40; 41, 42) with a determined illumination surface for subjecting a portion of said organic tissue to a light emission in at least one determined wavelength range; and a detection device (2) distant from said illumination device (1) for detecting the intensity of the light emission produced by said illumination device after propagation in said organic tissue (50), **characterized in that** said illumination device (1) includes an optical element forming a guide (10; 101, 102) coupled to said at least one light source (40; 41, 42) for guiding the light emission from said source by total internal reflection in a substantially parallel direction to the surface of the organic tissue to be illuminated (50) and for distributing said light emission into several determined illumination zones on the surface of said organic tissue (50) over a substantially broader area than the illumination surface of said light source (40; 41, 42).

**2.** Portable instrument for measuring a physiological quantity (3) arranged to be brought into contact with the surface of an organic tissue (50) including, essentially arranged in a same plane:

an illumination device (1) including at least one light source (40; 41, 42) with a determined illumination surface for subjecting a portion of said organic tissue to a light emission in at least one determined wavelength range; and a detection device (2) distant from said illumination device (1) for detecting the intensity of the light emission produced by said illumination device after propagation in said organic tissue (50), **characterized in that** said detection device (2) includes an optical element forming a guide (201, 202, 203) coupled to at least one photoreceptor (21, 22, 23) for picking up in several zones of the organic tissue surface (50) the light emission produced by the illumination device (1) after propagation in the organic tissue and for guiding said light emission by total internal reflection to said at least one photoreceptor (21, 22, 23).

**3.** Instrument according to claim 1, **characterized in that** said detection device (2) has a determined geometrical contour and **in that** each illumination zone is determined to be at a substantially constant distance from the closest point of said geometrical contour of the detection device.

**4.** Instrument according to claim 1, 2 or 3, **characterized in that** said optical element forming a guide has a branched and/or at least partially cellular structure.

**5.** Instrument according to claim 4, **characterized in that** said optical element forming a guide has an essentially honeycombed structure.

**6.** Instrument according to claim 1, **characterized in that** said detection device (2) includes one or several quasi-punctual photoreceptors (21, 22, 23) essentially arranged in the same plane as said optical element forming a guide (10) and **in that** said optical element forming a guide (10) has a structure whose geometry essentially coincides with an arc of a circle or a circle of predetermined radius around the position of each photoreceptor.

**7.** Instrument according to claim 1, **characterized in that** said optical element (10; 101, 102) is a solid optical guide provided with reflective micro-prismatic structures (60) for redirecting the light emission produced by said light source (40; 41, 42) in the direction of the organic tissue surface to be illuminated (50),
said optical element having a first face (10a), or lower face, directed towards said organic tissue to be illuminated (50), a second face (10b), or upper face, opposite said first face, and lateral faces (10c, 10d) joining said first and second faces, oriented substantially perpendicularly to the organic tissue surface to be illuminated,
each of said micro-prismatic structures (60) including at least a first facet (61; 62) arranged on said second face (10b) and/or said lateral faces (10c, 10d) to redirect said light emission through said first face (10a) in the direction of said organic tissue to be illuminated (50).

**8.** Instrument according to claim 2, **characterized in that** said optical element (201, 202, 203) is a solid optical guide provided with reflective micro-prismatic structures for redirecting the light emission emanating from the organic tissue (50) in the direction of the associated photoreceptor or photoreceptors (21, 22, 23), said optical element having a first face, or lower face, directed towards said organic tissue (50) from which the light emission originates, a second face, or upper face, opposite said first face, and lateral faces joining said first and second faces, oriented substantially perpendicularly to the organic tissue surface,
each of said micro-prismatic structures including at least a first facet arranged on said second face and/or said lateral faces to redirect said light emission emanating from the organic tissue in the direction of the associated photoreceptor or photoreceptors.

**9.** Instrument according to claim 7 or 8, **characterized in that** at least the first facet of each reflective micro-prismatic structure is coated with a reflective coating.

**10.** Instrument according to any one of claims 7 to 9, **characterized in that** the length of said micro-prismatic structures and/or the number thereof increases progressively as one moves away from said light source, respectively from the associated photoreceptor, along the optical path of the light emission in said optical element.

**11.** Instrument according to claim 1, **characterized in that** it includes a first light source for producing a light emission within a first determined wavelength range and a second light source for producing a light emission within a second determined wavelength range different from the first,
said optical element forming a guide being arranged for mixing the light emissions from said first and second light sources.

**12.** Instrument according to claim 1, **characterized in that** said detection device (2) includes an optical element forming a guide (201, 202, 203) coupled to at least one photoreceptor (21, 22, 23) for picking up in several zones of the organic tissue surface (50) the light emission produced by the illumination device (1) after propagation in the organic tissue and for guiding said light emission by total internal reflection towards said at least one photoreceptor (21, 22, 23).

**13.** Instrument according to any one of the preceding claims, **characterized in that** it is worn on the wrist and includes a case (30) provided with a back cover (35) arranged to come into contact with the user's skin and in which said illumination (1) and detection (2) devices are arranged.

**14.** Instrument according to claim 13, **characterized in that** said back cover (35) is provided with a rib (70) intended to abut on said organic tissue (50) to keep a space between the optical element forming a guide (10; 101, 102, 201, 202, 203) and the organic tissue.

**15.** Instrument according to claim 13, **characterized in that** at least a portion of the surface of said back cover (35) located between the illumination and detection devices is arranged so as not to reflect the light emission produced by said illumination.

**Patentansprüche**

**1.** Tragbares Instrument zum Messen einer physiologischen Größe (3), das so beschaffen ist, dass es in einen Kontakt mit der Oberfläche eines organischen Gewebes (50) gebracht wird und die folgenden Vorrichtungen, die im Wesentlichen in derselben Ebene angeordnet sind, umfasst:

eine Beleuchtungsvorrichtung (1), die wenigstens eine Lichtquelle (40; 41, 42) mit gegebener Beleuchtungsoberfläche umfasst, um einen Abschnitt des organischen Gewebes einer Lichtemission in wenigstens einem bestimmten Wellenlängenbereich auszusetzen; und
eine Erfassungsvorrichtung (2), die von der Beleuchtungsvorrichtung (1) beabstandet ist, um die Intensität der von der Beleuchtungsvorrichtung erzeugten Lichtemission nach der Ausbreitung in dem organischen Gewebe (50) zu erfassen,
**dadurch gekennzeichnet, dass** die Beleuchtungsvorrichtung (1) ein einen Leiter bildendes optisches Element (10; 101, 102) umfasst, das mit der wenigstens einen Lichtquelle (40; 41, 42) gekoppelt ist, um die Lichtemission dieser Quelle durch innere Totalreflexion in einer Richtung zu führen, die zu der zu beleuchtenden Oberfläche des organischen Gewebes (50) im Wesentlichen parallel ist, und um diese Lichtemission auf mehrere bestimmte Beleuchtungszonen auf der Oberfläche des organischen Gewebes (50), deren Flächeninhalt wesentlich größer ist als die Beleuchtungsoberfläche der Lichtquelle (40; 41, 42), zu verteilen.

2.  Tragbares Instrument zum Messen einer physiologischen Größe (3), das so beschaffen ist, dass es mit der Oberfläche eines organischen Gewebes (50) in Kontakt gebracht wird, und die folgenden Vorrichtungen, die im Wesentlichen in derselben Ebene angeordnet sind, umfasst:

eine Beleuchtungsvorrichtung (1), die wenigstens eine Lichtquelle (40; 41, 42) mit gegebener Beleuchtungsoberfläche aufweist, um einen Abschnitt des organischen Gewebes einer Lichtemission in wenigstens einem bestimmten Wellenlängenbereich auszusetzen; und
eine Erfassungsvorrichtung (2), die von der Beleuchtungsvorrichtung (1) beabstandet ist, um die Intensität der von der Beleuchtungsvorrichtung erzeugten Lichtemission nach der Ausbreitung in dem organischen Gewebe (50) zu erfassen,
**dadurch gekennzeichnet, dass** die Erfassungsvorrichtung (2) ein einen Leiter (201, 202, 203) bildendes optisches Element umfasst, das mit wenigstens einem Photoempfänger (21, 22, 23) gekoppelt ist, um in mehreren Zonen der Oberfläche des organischen Gewebes (50) die Lichtemission, die von der Beleuchtungsvorrichtung (1) erzeugt wird, nach der Ausbreitung in dem organischen Gewebe einzufangen und um diese Lichtemission durch innere Totalreflexion zu dem wenigstens einen Photoempfänger (21, 22, 23) zu leiten.

3.  Instrument nach Anspruch 1, **dadurch gekennzeichnet, dass** die Erfassungsvorrichtung (2) einen bestimmten geometrischen Umfang aufweist und dass jede Beleuchtungszone so bestimmt ist, dass sie sich in einem im Wesentlichen konstanten Abstand vom nächsten Punkt des geometrischen Umfangs der Erfassungsvorrichtung befindet.

4.  Instrument nach Anspruch 1, 2 oder 3, **dadurch gekennzeichnet, dass** das einen Leiter bildende optische Element eine verzweigte und/oder wenigstens teilweise zellenartige Struktur aufweist.

5.  Instrument nach Anspruch 4, **dadurch gekennzeichnet, dass** das einen Leiter bildende optische Element im Wesentlichen eine Bienenwabenstruktur aufweist.

6.  Instrument nach Anspruch 1, **dadurch gekennzeichnet, dass** die Erfassungsvorrichtung (2) einen oder mehrere quasi-punktförmige Photoempfänger (21, 22, 23) aufweist, die im Wesentlichen in derselben Ebene wie das einen Leiter bildende optische Element (10) angeordnet sind, und dass das einen Leiter bildende optische Element (10) eine Struktur aufweist, deren Geometrie im Wesentlichen mit einem Kreisbogen oder mit einem Kreis mit vorgegebenem Radius um die Position jedes Photoempfängers übereinstimmt.

7.  Instrument nach Anspruch 1, **dadurch gekennzeichnet, dass** das optische Element (10; 101, 102) ein massiver Lichtleiter ist, der mit reflektierenden Mikroprismenstrukturen (60) versehen ist, um die von der Lichtquelle (40: 41, 42) erzeugte Lichtemission in Richtung der zu beleuchtenden Oberfläche des organischen Gewebes (50) umzulenken,
wobei das optische Element eine erste Fläche (10a) oder untere Fläche, die zu dem zu beleuchtenden organischen Gewebe (50) gerichtet ist, eine zweite Fläche (10b) oder obere Fläche, die der ersten Fläche gegenüberliegt, und Seitenflächen (10c, 10d), die die erste und die zweite Fläche miteinander verbinden und im Wesentlichen senkrecht zu der beleuchtenden Oberfläche des organischen Gewebes orientiert sind, aufweist,
wobei jede der Mikroprismenstrukturen (60) wenigstens eine erste Facette (61; 62), die auf der zweiten Fläche (10b) und/oder den Seitenflächen (10c, 10d) ausgebildet ist, umfasst, um die Lichtemission durch die erste Fläche (10a) in Richtung des zu beleuchtenden organischen Gewebes (50) umzulenken.

8. Instrument nach Anspruch 2, **dadurch gekennzeichnet, dass** das optische Element (201, 202, 203) ein massiver Lichtleiter ist, der mit reflektierenden Mikroprismenstrukturen versehen ist, um die von dem organischen Gewebe (50) ausgehende Lichtemission in Richtung des oder der zugeordneten Photoempfänger (21, 22, 23) umzulenken, wobei das optische Element eine erste Fläche oder untere Fläche, die zu dem organischen Gewebe gerichtet ist, von dem die Lichtemission (50) stammt, eine zweite Fläche oder obere Fläche, die der ersten Fläche gegenüberliegt, und Seitenflächen, die die erste und die zweite Fläche miteinander verbinden und im Wesentlichen senkrecht zu der Oberfläche des organischen Gewebes orientiert sind, aufweist, wobei jede der Mikroprismenstrukturen wenigstens eine erste Facette, die auf der zweiten Fläche und/oder den Seitenflächen ausgebildet ist, umfasst, um die Lichtemission, die von dem organischen Gewebe ausgeht, in Richtung des oder der zugeordneten Photoempfänger umzulenken.

9. Instrument nach Anspruch 7 oder 8, **dadurch gekennzeichnet, dass** die wenigstens eine erste Facette jeder reflektierenden Mikroprismenstruktur mit einer reflektierenden Beschichtung überzogen ist.

10. Instrument nach einem der Ansprüche 7 bis 9, **dadurch gekennzeichnet, dass** die Länge der Mikroprismenstrukturen und/oder ihre Anzahl in dem Maß zunehmen, in dem sie von der Lichtquelle bzw. von dem zugeordneten Photoempfänger auf dem Lichtweg der Lichtemission in dem optischen Element weiter entfernt sind.

11. Instrument nach Anspruch 1, **dadurch gekennzeichnet, dass** es eine erste Lichtquelle, die eine Lichtemission in einem ersten bestimmten Wellenlängenbereich erzeugt, und eine zweite Lichtquelle, die eine Lichtemission in einem zweiten bestimmten Wellenlängenbereich erzeugt, der von dem ersten verschieden ist, umfasst, wobei das einen Leiter bildende optische Element so beschaffen ist, dass es die Lichtemissionen der ersten und der zweiten Lichtquelle mischt.

12. Instrument nach Anspruch 1, **dadurch gekennzeichnet, dass** die Erfassungsvorrichtung (2) ein einen Leiter bildendes optisches Element (201, 202, 203) umfasst, das mit wenigstens einem Photoempfänger (21, 22, 23) gekoppelt ist, um in mehreren Zonen der Oberfläche des organischen Gewebes (50) die von der Beleuchtungsvorrichtung (1) erzeugte Lichtemission einzufangen, nachdem sich diese in dem organischen Gewebe ausgebreitet hat, und um diese Lichtemission durch innere Totalreflexion zu dem wenigstens einem Photoempfänger (21, 22, 23) zu führen.

13. Instrument nach einem der vorhergehenden Ansprüche, **dadurch gekennzeichnet, dass** es am Handgelenk getragen wird und ein Gehäuse (30) aufweist, das mit einem Boden (35) versehen ist, der so beschaffen ist, dass er mit der Haut des Trägers in Kontakt gelangt und in dem die Beleuchtungsvorrichtung (1) und die Erfassungsvorrichtung (2) angeordnet sind.

14. Instrument nach Anspruch 13, **dadurch gekennzeichnet, dass** der Boden (35) mit einem Steg (70) versehen ist, der dazu bestimmt ist, sich auf dem organischen Gewebe (50) abzustützen, um zwischen dem einen Leiter bildenden optischen Element (10; 101, 102; 201, 202, 203) und dem organischen Gewebe einen Zwischenraum aufrecht zu erhalten.

15. Instrument nach Anspruch 13, **dadurch gekennzeichnet, dass** wenigstens ein Teil der Oberfläche des Bodens (35), der sich zwischen der Beleuchtungsvorrichtung und der Erfassungsvorrichtung befindet, so beschaffen ist, dass er die von der Beleuchtungsvorrichtung erzeugte Lichtemission nicht reflektiert.

# Fig.1

## Fig.1a

## Fig.1b

Fig.2a

Fig.2b

# Fig.3

# Fig.4

## Fig.5

# Fig. 6

# Fig.7

Fig.8

Fig.9

# Fig.10

intensité locale moyenne

distance optimale

taux de modulation (%)

intensité modulée locale

distance par rapport à la source